# FASCICULE DE BREVET EUROPEEN

(11) **EP 1 576 191 B1**
(45) Date de publication et mention de la délivrance du brevet: **22.11.2006**
(21) Numéro de dépôt: 03799647.7
(22) Date de dépôt: 19.12.2003
(51) Int. Cl.: C12Q 1/68

(54) **PROCEDE D'EVALUATION QUANTITATIVE DES CAPACITES GLOBALES ET SPECIFIQUES DE REPARATION DE L'ADN D'AU MOINS UN MILIEU BIOLOGIQUE, AINSI QUE SES APPLICATIONS.**
VERFAHREN ZUR QUANTITATIVEN EVALUIERUNG DER GESAMTEN UND SPEZIFISCHEN FÄHIGKEITEN ZUR DNS-REPARATUR MINDESTENS EINES BIOLOGISCHEN MITTELS, UND DESSEN ANWENDUNGEN
METHOD FOR THE QUANTITATIVE ASSESSMENT OF GLOBAL AND SPECIFIC DNA REPAIR CAPACITIES OF AT LEAST ONE BIOLOGICAL MEDIUM, AND THE APPLICATIONS THEREOF

(30) Priorité: 20.12.2002 FR 0216435
(43) Date de publication de la demande: 21.09.2005
(73) Titulaire: COMMISSARIAT A L'ENERGIE ATOMIQUE, 75015 Paris (FR)
(72) Inventeur: SAUVAIGO, Sylvie, F-38320 HERBEYS (FR)
(74) Mandataire: Vialle-Presles, Marie José
(86) Numéro de dépôt international: PCT/FR2003/003816
(87) Numéro de publication internationale: WO 2004/059004

(56) Documents cités:
- WO-A-99/49080
- US-A1- 2002 022 228
- US-B1- 6 309 838
- WOOD R D ET AL: "COMPLEMENTATION OF THE XERODERMA PIGMENTOSUM DNA REPAIR DEFECT IN CELL-FREE EXTRACTS" CELL, CELL PRESS, CAMBRIDGE, NA, US, vol. 53, 8 avril 1988 (1988-04-08), pages 97-106, XP002054959 ISSN: 0092-8674 cité dans la demande

## Description

La présente invention est relative à un procédé d'évaluation quantitative des capacités globales et spécifiques de réparation de l'ADN d'un milieu biologique, en évaluant les capacités d'excision/resynthèse dudit milieu ainsi qu'à ses applications.

L'ADN est sans cesse soumis à des agressions endogènes ou exogènes conduisant à la formation de lésions de bases ou de sucres.

Ces lésions incluent :
- les lésions des bases puriques ou pyrimidiques : lésions oxydatives induites par le métabolisme cellulaire, et par photosensibilisation ; lésions par formation d'adduits chimiques, qui résultent de l'action nocive de nombreux agents géno-toxiques tels que les hydrocarbures polycycliques contenus dans les produits de combustion ; lésions par formation de méthéno-bases ou d'éthéno-bases.
- les lésions de la structure de la double hélice de l'ADN : formation de pontages intrabrin (entre deux bases adjacentes d'un même brin) ou interbrins (entre deux bases situées sur les brins homologues), généralement provoqués par les ultraviolets (formation de ponts entre pyrimidines, qui deviennent dimériques) ou les antitumoraux bifonctionnels, tels que le cisplatine et les agents intercalants, qui forment des liaisons covalentes stables ente les bases portées par les brins opposés.
- lésions spontanées, du fait que l'ADN est une molécule partiellement instable : désaminations ou dépurinations spontanées.
- lésions par rupture simple-brin ou double-brin : produites par des agents tels que les radiations ionisantes et par l'action des radicaux libres.
- lésions des sucres : la destruction d'un désoxyribose entraîne une rupture des liaisons phosphodiesters au niveau du site lésé, suivie d'une rupture du brin.

La diversité des lésions induites est illustrée par l'analyse des photoproduits stables détectés à la suite d'une irradiation par les UV C : à côté des dimères de pyrimidine dus à la formation d'un noyau cyclobutanique, il se forme entre deux pyrimidines adjacentes, des pyrimidines (6-4) pyrimidones. La proportion relative des dimères de pyrimidine et de produit (6-4) varie de 10 à 4 pour 1. Leur efficacité respective dans l'effet létal et dans l'effet mutagène des ultraviolets est également différente : les dimères ont un rôle cytotoxique plus important que les produits (6-4), alors que l'inverse est vrai pour l'effet mutagène. De même, les radiations ionisantes, (rayons y du cobalt 60, par exemple) produisent simultanément des ruptures simple-brin ou double-brin (approximativement dans un rapport de 9 à 1), de nombreux produits d'addition des bases, des pertes de bases et aux fortes doses, des pontages entre ADN et protéines adjacentes (chromosomiques par exemple). On dénombre en moyenne une rupture de brin pour une base modifiée. Le rôle prédominant de la rupture double-brin dans l'effet cytotoxique des radiations est accompagné d'un effet mutagène dû aux altérations des bases.

Ces différents types de lésions peuvent être créés sur de l'ADN isolé. Par exemple, des lésions de type photoproduits (6-4) et dimères de pyrimidine de type cyclobutane sont induites par irradiation UVC (Hoeijmaker et al., *Mutation Res.,* 1990, 236, 223-238) ; des lésions de type oxydatif sont induites par la réaction de Fenton en présence de peroxyde d'hydrogène et de fer (Elliot et al., *Free Rad. Biol. Med*., 2000, 1438-1446). Un autre moyen de préparer de l'ADN modifié consiste à manipuler des plasmides par des techniques de biologie moléculaire et d'y insérer un oligonucléotide obtenu par synthèse chimique et contenant une lésion d'intérêt (Biade et al., *J. Biol. Chem.,* 1997, 273, 898-902).

Tous les organismes vivants disposent de systèmes de réparation de l'ADN, destinés à maintenir l'intégrité de leur génome.

Parmi ces systèmes de réparation, deux ont pour fonction d'éliminer les bases modifiées de l'ADN : il s'agit du système de Réparation par Excision de Bases (REB) et du système de Réparation par Excision de Nucléotides (REN) :
- Le système REB est plus spécifiquement dédié à la réparation des petites lésions de l'ADN telles que les dommages oxydatifs, les sites abasiques, les fragmentations de base, les méthylations de bases, les éthéno-bases, etc.
- Le système REN prend en charge les lésions volumineuses induisant une distorsion de la double-hélice d'ADN telles que les adduits acétylaminofluorène, cisplatine et psoralène de l'ADN, les dimères issus de l'irradiation UV B et UV C de l'ADN, les lésions covalentes formées entre une base de l'ADN et une autre molécule, etc. (Sancar et al., *Annu. Rev. Genetics,* 1995, 29, 69-105).

Ces différents systèmes de réparation présentent des caractéristiques communes et en particulier les étapes suivantes :
- reconnaissance de la ou des lésions par des protéines appartenant au système de réparation,
- excision de la lésion et éventuellement des nucléotides adjacents,
- resynthèse par les polymérases du milieu, des nucléotides manquants.
- la réparation se termine généralement, par la ligation du brin néoformé avec le brin d'ADN existant.

Dans tous ces cas, ce processus comprend, l'élimination du nucléotide modifié et l'incorporation en remplacement dans la chaîne d'ADN, d'au moins un nucléotide triphosphate présent dans le milieu de réparation.

Il faut cependant noter que les systèmes de réparation, surtout chez les eucaryotes, sont très complexes et de nombreuses variantes à ce schéma simpliste existent (réparation globale, réparation associée à la transcription de l'ADN, réparation associée à la réplication de l'ADN, etc). Certaines protéines sont impliquées dans plusieurs systèmes de réparation simultanément, d'autres sont spécifiques d'un seul système, certaines sont inductibles par des facteurs cellulaires ou externes, d'autres ont une expression ubiquitaire et constante.

Pour la suite de la description :

On appelle « substrat » tout ADN susceptible de subir une réaction de réparation en présence d'extrait cellulaire et par extension les lésions de l'ADN.

On appelle « milieu biologique » ou « extrait cellulaire » une préparation biologique purifiée ou non, susceptible de contenir au moins une activité enzymatique liée à la réparation de l'ADN.

On peut généralement associer une lésion aux protéines spécifiques chargées de sa réparation dans l'ADN. Des différences existent selon les espèces ; chez les procaryotes comme *Escherichia coli,* les enzymes sont moins spécifiques, tandis que chez l'homme, on remarque une association beaucoup plus stricte lésion-enzyme de réparation spécifique surtout dans le systèmes REB. Par exemple, Lindahl et Wood (Science, 1999, 286, 1897-1905) décrivent les enzymes du système REB les plus importantes chez l'homme ainsi que les lésions qui leur sont associées. Par exemple, chez l'homme, la protéine OGG1, qui est une glycosylase appartenant au système REB est associée à la réparation de la 8-oxo-2'désoxyguanosine. Chez *Escherichia coli,* la formamidopyrimidine-ADN-N glycosylase répare cette même lésion mais plus généralement aussi les bases purines oxydées (Seeberg et al.., TIBS, 1995, 20, 391-397). La protéine humaine ANPG est l'équivalente de la protéine bactérienne AlkA. Ces enzymes n'ont cependant pas les mêmes affinités pour leurs substrats et possèdent des constantes de vitesse d'excision différentes (Laval et al., Mut. Res., 1998, 402, 93-102). Plus d'une quarantaine de lésions différentes prises en charge par le système REB peuvent avoir des conséquences biologiques importantes et négatives si elles ne sont pas réparées. On considère que les enzymes chargées de leur réparation ont un rôle anti-tumoral important. On voit que la connaissance précise de leurs spécificités de substrat est très importante.

Des tests de capacités de réparation cellulaire ont été développés et peuvent être classés en deux catégories : tests *in vitro* qui nécessitent l'emploi d'extraits cellulaires actifs et systèmes *in vivo* ou semi *in vivo* réalisés sur cellules vivantes.

### I. Méthodes basées sur la mesure de l'activité d'excision/resynthèse

A. La plupart des tests *in vitro* ont été développés sur la base des expérimentations décrites par Wood et al. (*Cell*, 1988, 53, 97-106 et *Biochemistry,* 1989, 26, 8287-8292), qui évaluent l'étape d'excision/resynthèse de la réparation.

De manière plus précise, ce test comporte l'utilisation d'un ADN plasmidique dans lequel des lésions sont introduites (par irradiation UV : formation de dimères de pyrimidine, pontages ; par action de la DNase I : coupure ou rupture simple brin) ; l'ADN ainsi modifié est incubé à 30°C en présence d'une préparation de réparation comportant au moins : l'extrait cellulaire à évaluer, un nucléotide triphosphate marqué en position alpha par du ³²P et de l'ATP. Les enzymes contenues dans l'extrait incisent l'ADN plasmidique et éliminent les lésions. De l'ADN est synthétisé de *novo* en remplacement des nucléotides éliminés. Le nucléotide radioactif introduit dans le milieu est incorporé dans l'ADN pendant cette synthèse. Après isolement du plasmide réparé par électrophorèse sur gel d'agarose, on mesure la quantité de radioactivité incorporée, qui est proportionnelle au taux de réparation du substrat. La méthode de préparation de l'extrait cellulaire et les conditions de réaction interviennent dans la qualité de la réparation. En particulier, il semble que l'on obtienne le meilleur rendement de réparation avec des extraits de cellules entières du type de ceux utilisés pour les transcriptions *in vitro,* alors que des extraits cytosoliques du type de ceux utilisés pour promouvoir la réplication plasmidique à partir d'une origine SV40 ainsi que d'autres extraits cellulaires bruts présentent une activité nucléasique, qui ne permet pas une interprétation correcte de la réparation.

Dans les conditions établies par Wood et al., la spécificité de la réaction pour ce qui concerne l'ADN irradié est plus importante en présence de concentration en KCl de l'ordre de 40-100 mM. En outre, la réplication de l'ADN irradié, qui a lieu pendant la réparation est fortement dépendante de la présence d'ATP et d'un système de régénération de l'ATP (phosphocréatine + créatine phosphokinase), en vue du maintien d'un taux constant d'ATP, ce maintien étant plus spécifiquement associé à l'étape d'incision de la réparation. Une telle dépendance n'est pas rencontrée par exemple dans les cas de réparation de rupture de brin. Un échantillon contrôle, constitué du même plasmide non modifié est utilisé simultanément dans les mélanges réactionnels.

Le test de Wood et al. nécessite l'emploi de marqueurs radioactifs, ce qui impose des contraintes qui limitent la réalisation de cette méthode dans des tests de routine ; en outre, ce test ne présente pas des qualités de simplicité et de praticabilité suffisantes, pour son utilisation en routine.

La méthode de Wood et al. a été proposée dans des essais de caractérisation d'extraits provenant de cellules établies à partir de patients atteints de *xeroderma pigmentosum* (Satoh et al., *Proc. Natl. Acad. Sci.* USA, 1993, 90, 6335-6339 ; Jones et al., *Nucl. Acids Res.,* 1992, 20, 991-995 ; Robins et al., EMBO J., 1991, 10, 3913-3921). Le *xeroderma pigmentosum* est une maladie multigénique, multiallélique, autosomale et récessive. Les cellules provenant de patients atteints par cette maladie sont très sensibles aux ultraviolets et présentent des défauts dans la réparation de l'ADN. Huit gènes sont impliqués dans les différents groupes de complémentation de cette maladie : XPA à XPG et le groupe variant XPV. Chaque groupe possède des caractéristiques différentes relatives à la réparation de l'ADN et notamment aux différents sous-types de REN. Les lésions de l'ADN, qu'elles appartiennent à la catégorie des petites lésions ou des lésions volumineuses, sont réparées de manière différente, selon le groupe de complémentation.

D'autres maladies de la réparation (Syndrome de Cockayne, *Ataxia Telangectasia*) possèdent également des caractéristiques propres de réparation et ont été étudiées par la méthode de Wood et al..

B. Dans différentes publications, l'équipe de B. Salles et P. Calsou (Biochimie 1995, 77, 796-802 ; Anal. Biochem. 1995, 232, 37-42) décrit un procédé de détection des lésions de l'ADN en réalisant des réactions d'excision/resynthèse sur un plasmide fixé dans des puits de microplaques. Le plasmide est adsorbé dans des puits de microplaques puis modifié *a posteriori* par des agents chimiques. Des extraits cellulaires sont rajoutés dans les puits ainsi que des nucléotides triphosphate marqués à la digoxygénine. Le marqueur est incorporé dans l'ADN, s'il y a eu excision de lésions, lors de l'étape de resynthèse. Le marqueur est ensuite révélé dans chaque puits par un anticorps couplé à la phosphatase alcaline. On rajoute, dans chaque puits, un substrat qui devient luminescent après déphosphorylation par la phosphatase alcaline. Le signal luminescent, émis au niveau de chaque puits, est mesuré. Il est proportionnel au taux d'incorporation du marqueur.

Cette méthode est également décrite dans la Demande Internationale WO 96/28571, dont les Inventeurs appartiennent également à l'équipe de B. Salles et P. Calsou, et qui décrit un procédé de détection qualitative et quantitative de lésions de l'ADN dans lequel, de l'ADN lésé est fixé sur un support solide et une composition comprenant un extrait cellulaire à tester et contenant des marqueurs est mise en contact avec ledit ADN lésé (préalablement ou postérieurement à la fixation sur ledit support solide). Ils considèrent que leur méthode permet de traiter simultanément un nombre d'échantillons important ; si l'on se réfère aux exemples, la réparation en présence d'un extrait cellulaire se fait dans un milieu réactionnel de 50 µl, à partir d'un extrait comprenant 150 µg de protéines, 50 mM de KCl, 5 mM de chlorure de magnésium, du DTT, de la phosphocréatine, de la phosphocréatine kinase et différents dNTP, l'un d'entre eux étant marqué à la digoxygénine. La réparation est obtenue après 3 heures d'incubation à 30°C et les puits sont lavés avec une solution de lavage comprenant un tampon phosphate avec un sel auquel on rajoute un tensioactif non ionique (Tween 20) dans une proportion de 0,05 et 0,15 % (composition préférée : tampon phosphate 10 mM, NaCl 137 mM et Tween 20 0,1 %). Il est précisé que ce test est d'une grande sensibilité, dans la mesure où la détection est opérée sur 40 ng d'ADN au lieu de 200 ou 300 ng, dans le cadre d'un test en solution.

Le test de l'équipe de B. Salles et P. Calsou propose essentiellement de modifier le plasmide après fixation sur le support solide. Or, on sait que parmi les lésions créées par de nombreux agents chimiques ou physiques, on trouve les cassures de chaînes. Or, ces cassures sont réparées très rapidement et efficacement par des extraits cellulaires actifs. Avec ce test, il est donc impossible de différencier la réparation des cassures et la réparation des autres lésions. La réparation des cassures peut même masquer la réparation des autres lésions et interférer avec les signaux attribués à la réparation d'autres lésions de l'ADN. Il s'agit donc d'un système qui permet la détection d'un effet global, sans identifier les lésions reconnues par les systèmes de réparation ; en outre ce procédé n'a pas pour but de détecter et quantifier l'activité des protéines impliquées dans la réparation de l'ADN, mais d'identifier la présence de lésions sur l'ADN traité.

### II. Méthodes basées sur l'évaluation de l'étape d'incision/excision

Des variantes de la méthode de Wood et al. ont été proposées et permettent notamment de mesurer uniquement l'activité d'incision des lésions :

**A.** Redaelli et al. (Terat. Carcinog. Mut., 1998, 18, 17-26) décrivent une méthode dans laquelle le plasmide est incubé directement avec l'extrait sans les nucléotides triphosphate. Les coupures dans le plasmide super enroulé provoquent un changement dans la vitesse de migration dans le gel d'agarose pendant l'électrophorèse. Le plasmide super enroulé migre plus vite que le plasmide incisé, du fait de sa conformation. Les bandes correspondant aux différentes formes du plasmide sont quantifiées ; la quantité de la forme incisée est corrélée à l'activité d'incision des lésions du plasmide, contenues dans l'extrait.

De manière plus précise, cet article étudie l'action d'incision de l'AP-endonucléase, qui intervient sur un site abasique, obtenu après action d'une glycosylase spécifique de la modification à réparer (alkylation, désamination hydrolytique, oxydation, mésappariement), en clivant le lien désoxyribosique phosphodiester en 3' ou en 5' de ce site abasique. Dans cet article l'activité AP-endonucléase est plus spécifiquement étudiée sur un extrait brut de lymphocytes humains. L'extrait (80 µl) est incubé d'une part avec un plasmide non endommagé (contrôle) et d'autre part avec un plasmide dépuriné. La quantification de l'activité de l'AP-endonucléase se révèle ainsi possible, dans la mesure où l'activité d'incision est dépendante du dommage et sensible à l'EDTA.

**B.** l'équipe de P. Calsou et B. Salles *(Biochem. Biophys. Res. Com.,* 1994, 202, 788-795) propose une autre approche de la mesure spécifique de l'activité d'incision des lésions d'un plasmide. Ils introduisent dans le milieu de réparation un inhibiteur spécifique des polymérases eucaryotes, l'aphidicoline, afin d'empêcher la resynthèse par les polymérases endogènes des fragments d'ADN normaux après la première étape d'excision. Une polymérase procaryote exogène est mélangée au milieu réactionnel en quantité équivalente pour tous les tubes. Des différences dans les résultats obtenus sont ainsi le reflet de l'étape d'excision des lésions et non pas de la resynthèse des fragments d'ADN excisés.

**C.** Une autre méthode basée sur une modification du test des comètes (électrophorèse sur gel en milieu alcalin d'une seule cellule) permet également de mesurer l'activité d'incision. Elle a été développée par Collins et al. (Mutagenesis, 2001, 16, 297-301). Des lésions oxydatives sont introduites sur l'ADN génomique par photosensibilisation de cellules HeLa en présence de lumière visible. Les cellules sont ensuite incorporées dans un gel d'agarose étalé sur une lame de microscope, puis les membranes cellulaires et les protéines sont éliminées par lyse ménagée. Les nucléoïdes isolés dans le gel sont incubés en présence d'extraits cellulaires qui sont actifs pour la première étape d'incision des lésions. Les lames sont ensuite soumises à une électrophorèse en milieu alcalin. La présence de coupure induit une migration de l'ADN plus rapide que l'ensemble nucléoïde. La pelote d'ADN prend alors l'aspect d'une comète, l'ADN intègre étant dans la tête et l'ADN contenant des coupures dans la queue de la comète. Le pourcentage d'ADN dans la queue de la comète déterminé par des logiciels spécialisés, est directement corrélé à l'activité d'incision contenue dans les extraits utilisés pour les lésions considérées. Ce test a été appliqué à la mesure des activités d'excision des dommages oxydatifs dans des extraits provenant de lymphocytes humains. Par rapport à la méthode décrite par Redaelli et al., 1998, qui mesure la coupure des plasmides, la méthode de Collins et al. met en oeuvre la méthode des comètes, pour estimer les coupures de brin et considère que cette variante est d'une part significativement plus sensible (détection d'environ 0,2 à 2 coupures par 10⁹ daltons) et d'autre part économiquement intéressante (économies sur le matériel utilisé), dans la mesure où le volume de mélange réactionnel (ADN inclus dans un gel) est seulement de 50 µl et que l'on obtient suffisamment de matériel à tester à partir de 10 ml de sang (possibilité de réaliser plusieurs incubations).

**D.** La Demande Internationale WO 01/90408 décrit un procédé de détection et de caractérisation d'activités de protéines impliquées dans la réparation de lésions de l'ADN.

De manière plus précise cette méthode comprend la fixation sur un support solide d'au moins un ADN endommagé comportant au moins une lésion connue ; cet ADN endommagé est ensuite soumis à l'action d'une composition de réparation contenant ou non au moins une protéine intervenant pour la réparation de cet ADN endommagé et la détermination de l'activité de cette protéine pour la réparation en mesurant la variation d'un signal émis par un marqueur qui se fixe sur ou s'élimine du support lors de l'étape précédente.

Ce système, qui est mis en oeuvre avec un ADN endommagé qui se présente sous la forme d'un oligonucléotide de 15 à 100 bases ou d'un polynucléotide de 100 à 20 000 bases permet ainsi d'accéder à une information plus globale que les autres tests, puisque l'excision de plusieurs substrats peut être suivie simultanément.

Cependant, ce procédé concerne la mise en évidence d'activités d'incision de lésions de l'ADN. Il est ainsi limité à la caractérisation de l'étape d'excision de lésions pouvant être introduites dans des oligonucléotides de synthèse. D'autre part, bien qu'il donne des informations importantes sur les activités d'excision, il n'est pas adapté et ne décrit pas une quantification précise des activités enzymatiques d'**excision/resynthèse** de l'ADN.

Outre les inconvénients particuliers à chaque technique, rapportés ci-dessus, ces différentes méthodes présentent également les inconvénients suivants :
- Tous les tests décrits ci-dessus nécessitent l'utilisation de quantités de matériel biologique et notamment d'extraits cellulaires supérieurs à 10 µl : le volume réactionnel généralement utilisé est de 50 µl comportant de 10 à 40 µl d'extrait pour une quantité de protéines d'environ 100 µg. Les extraits sont longs à préparer, la quantité de cellules disponibles est souvent faible ; ce qui limite le nombre de tests réalisables.
- Quelle que soit la méthode de détection utilisée et que le test soit effectué en solution ou sur support, tous les systèmes décrits donnent une information sur la réparation point par point limitée à un substrat donné pour une fraction aliquote d'extrait ; en effet, dans les essais de détermination de capacités de réparation tels que proposés par Wood et al., par exemple, chaque test est réalisé individuellement dans un tube, c'est-à-dire qu'une réaction se fait en présence d'un plasmide donné et d'un extrait donné. Pour chaque extrait à tester, on compare le taux d'incorporation du marqueur dans le plasmide par rapport aux taux d'incorporation de marqueur obtenu dans un substrat préparé à l'identique en présence de l'extrait contrôle. L'extrait de contrôle de référence est généralement préparé à partir de cellules caractérisées transformées par EBV ou SV40. Il en est de même dans la plupart des autres variantes de la méthode de Wood et al., décrites ci-dessus.
- Les tests étant relativement lourds à réaliser et nécessitant la disponibilité de grandes quantités de matériel biologique, les expérimentateurs limitent le nombre de substrats utilisés et le nombre d'extraits biologiques testés.
- Les lésions introduites sur les plasmides ne sont ni mesurées ni quantifiées. Les auteurs, utilisant le test développé par Wood et al., éliminent uniquement les plasmides ayant perdu leur forme super enroulée afin d'éliminer l'ADN comportant des cassures de chaînes. Les informations obtenues sont très partielles et insuffisantes pour définir et caractériser précisément les capacités de réparation d'un milieu biologique donné.

En conséquence, la Demanderesse s'est donnée pour but de pallier les inconvénients de l'art antérieur, notamment en proposant un procédé qui permet de caractériser et de quantifier les activités enzymatiques **d'excision/resynthèse** de réparation de l'ADN dans des extraits biologiques d'une façon rapide, précise, miniaturisée et efficace et ce sans l'utilisation de solutions de réparation de contrôle.

La présente invention a pour objet un procédé d'évaluation quantitative des capacités globales et spécifiques de réparation de l'ADN d'au moins un milieu biologique, lequel procédé est caractérisé en ce qu'il comporte les étapes suivantes :
(a) la préparation d'une gamme de plasmides comportant chacun des lésions distinctes de l'ADN, par traitement indépendant desdits différents plasmides par au moins un agent physique et/ou chimique et récupération de la fraction super enroulée de chacun desdits plasmides ; la sélection des fractions super enroulées permet d'exclure les cassures de brin et d'éviter l'action des nucléases,
(b) la caractérisation des lésions présentes sur chacun des plasmides de ladite gamme de plasmides,
(c) le dépôt des différents plasmides de ladite gamme de plasmides et d'au moins un plasmide super enroulé contrôle sans lésions sur un support solide unique, selon un schéma A préétabli, pour former un support fonctionnalisé divisé en zones distinctes A₁ à Aₓ, x correspondant à un nombre entier égal au nombre de milieux biologiques à tester simultanément, chaque zone A₁ à Aₓ comprenant ladite gamme de plasmides ; en conséquence, les différentes préparations de plasmides de ladite gamme de plasmides modifiés sont déposée sur un même support solide en des endroits définis et repérés. Au moins un contrôle constitué d'un plasmide super enroulé sans lésions de l'ADN est déposé conjointement.
(d) l'incubation dudit support fonctionnalisé obtenu à l'étape (c) avec différentes solutions de réparation comprenant chacune au moins un milieu biologique susceptible de contenir des activités enzymatiques de réparation, de l'ATP, un système de régénération de l'ATP, un nucléotide triphosphate marqué et tout autre composant nécessaire à l'activité des enzymes de réparation présentes dans ledit milieu biologique, de préférence à une température de 30°C pendant 1 à 5 heures, de préférence pendant 3 heures chacune desdites solutions de réparation étant déposée, préalablement à ladite incubation, dans chacune desdites zones A₁ à Aₓ distinctes et préétablies dudit support fonctionnalisé,
(e) au moins un lavage dudit support fonctionnalisé,
(f) la mesure directe ou indirecte du signal produit par le marqueur incorporé dans l'ADN lors de la réaction de réparation de l'étape (d), dans chacune desdites zones A₁ à Aₓ distinctes et préétablies,
(g) l'enregistrement et la quantification du signal correspondant à chaque dépôt de plasmide dans chaque zone A₁ à Aₓ et
(h) la détermination du rapport des signaux des plasmides comportant les lésions par rapport au plasmide contrôle déposé conjointement.

Un tel procédé selon l'invention présente un certain nombre d'avantages :
- Il permet de détecter un effet global, en identifiant les différentes lésions, du fait de la possibilité d'évaluer simultanément la réparation de différents types de lésions.
- Il permet la détermination des capacités d'excision et/ou d'excision/resynthèse d'un extrait biologique sans recourir à la comparaison avec un milieu biologique contrôle. En effet, les résultats obtenus par la réalisation du procédé avec un seul échantillon d'extraits biologiques sont suffisants pour attribuer une efficacité de réparation à l'extrait par rapport à des lésions précises et quantifiées.
- Il est particulièrement bien adapté à l'étude de milieux biologiques divers et constitue un bon reflet de la situation *in vivo.*
- Le procédé selon l'invention permet de « cartographier » un milieu biologique donné pour ses activités enzymatiques de réparation de l'ADN. Il permet d'identifier un extrait biologique en fonction de la carte obtenue.
- Il permet de déterminer les protéines de la réparation déficientes ou partiellement déficientes dans un extrait biologique donné et donc de servir de test diagnostique.
- Le procédé selon l'invention permet en outre la comparaison des performances de différents extraits biologiques pour la réparation des lésions de l'ADN.
- Il n'utilise pas d'isotope radioactif.
- Etant miniaturisé, il permet d'obtenir de nombreuses informations à partir de très faibles quantités de matériel biologique.
- Il est automatisable.

Selon un mode de mise en oeuvre avantageux dudit procédé, les plasmides préparés à l'étape (a) sont choisis parmi ceux qui possèdent une forme double-brin super enroulée (pBR322, M13, pUC, etc...).

La forme super enroulée du plasmide contrôle est obtenue par purification en utilisant des techniques connues, comme par exemple, les kits de purification de plasmide Qiagen. Il est préférable, en outre, de limiter la présence de formes non désirées de plasmide en effectuant d'autres étapes de purification, comme par exemple la centrifugation sur chlorure de césium et/ou sur gradient de saccharose.

Selon un autre mode de mise en oeuvre avantageux de l'étape (a) dudit procédé, les différents agents physiques, biologiques ou chimiques aptes à induire une lésion de l'ADN sont choisis parmi ceux qui induisent de préférence : la formation d'une lésion unique, la formation d'un nombre limité de lésions ou la formation de différentes lésions appartenant à une même famille.

On peut citer par exemple comme familles de lésions : les lésions oxydatives, les photoproduits induits par les ultraviolets B ou C, les adduits chimiques, les éthéno-bases, les sites abasiques et les cassures d'ADN.

Les agents physiques et chimiques sont par exemple choisis parmi ceux qui fonctionnent principalement :
- par un mécanisme de photosensibilisation type II : l'oxygène singulet a pour cible principale la guanine ; dans ce cas, la lésion très majoritaire formée est la 8-oxoguanine (Ravanat et al., Chem. Res. Tox., 1995, 8, 379-3 88).
- par un mécanisme de photosensibilisation de type I ou par un mécanisme libérant le radical OH° ; dans ce cas, les lésions de l'ADN obtenues sont des lésions oxydatives ; ces lésions touchent, de façon équivalente, les bases puriques et les bases pyrimidiques de l'ADN. On peut citer parmi ces lésions, la 8-oxoguanine, les glycols, de thymine, la fapy-guanine, la fapy-adénine, l'hydroxyméthyl-uracile, la 5-hydroxyméthyl-cytosine, la formyl-uracile (Cadet et al., Rev. Physiol. Biochem. Pharm., 1997, 31, 1,87).
- par un mécanisme de transfert d'énergie de type triplet-triplet ; dans ce cas, les principales lésions formées sont des dimères de pyrimidine de type cyclobutane (Costalat et al., Photochem. Photobiol., 1990,51, 255-262).
- en libérant de l'énergie absorbée directement par les bases de l'ADN comme les ultraviolets B ou C. Les liaisons formées sont les dimères de pyrimidine de type cyclobutane, les photoproduits (6-4) et l'isomère de Valence Dewar (Douki et al., J. Biol. Chem., 2000,275, 11678-11685).
- en libérant de l'oxygène singulet. Ces agents appartiennent, par exemple, à la famille des endoperoxydes. La lésion formée est dans ce cas la 8-oxoguanine (Ravanat et al., J. Biol. Chem., 2001, 276, 40601-40604).

Les agents chimiques sont choisis parmi ceux qui induisent des modifications de bases connues appartenant entre autres à la famille des carcinogènes. On peut citer par exemple : l'acétyl-amino fluorène (Hess et coll, 1996, Nucleic Acid Res.24, 824-828), le cis-platine (Pasheva et coll, 2002, Int. J. Biochem. Cell Biol., 34, 87-92) Int, les benzo-pyrène (Laws et coll, 2001, Mut. Res. ; 484, 3-18), le psoralène (Zhang et coll, Mol. Cell. Biol., 2002, 22, 2388-2397), le chloroacétaldéhyde (CAA-Wang et coll, 2002, 13, 1149-1157), le tamoxifène (Dasaradhi et coll, 1997, Chem. Res. Tox., 10, 189-196) et le trans, trans-2, 4-décadiénal (DDE-Carvalho et coll, 1998, Chem. Res. Tox., 11, 1042-1047).

Selon encore un autre mode de mise en oeuvre avantageux de l'étape (a) dudit procédé, différents agents sont mis en oeuvre sur chaque plasmide de ladite gamme de plasmides.

Selon un mode de mise en oeuvre avantageux de l'étape (b) dudit procédé, la caractérisation des lésions comprend (i) le prélèvement d'une fraction de chaque plasmide lésé, (ii) la digestion de chacune desdites fractions par des enzymes libérant les nucléosides de l'ADN, puis (iii) l'analyse du résultat de la digestion en utilisant une combinaison de techniques séparatives couplées à une technique analytique quantitative.

Selon une disposition avantageuse de ce mode de mise en oeuvre, la digestion est mise en oeuvre à l'aide d'au moins l'une des enzymes suivantes : la phosphodiestérase de rate de veau, la nucléase P1, la phosphodiestérase de venin de serpent, et la phosphatase alcaline (Douki et al., J. Biol. Chem., 2000,275, 11678-11685).

Selon une autre disposition avantageuse de ce mode de mise en oeuvre, le résultat de la digestion enzymatique est analysé par l'une des techniques suivantes : Chromatographie Liquide Haute Performance (CLHP) couplée à la spectrométrie de masse en Tandem (Douki et coll, 2000, J. Biol. Chem., 275, 11678-11685 ; Sauvaigo et coll, 2001, Photochem. Photobiol., 73, 230-237 ; Frelon et coll., Chem. Res. Tox., 2000, 13, 1002-1010), par CLHP couplée à la Chromatographie Gazeuse (Wang et coll, 2000, 13, 1149-1157 ; Pouget et coll, 2000, Chem. Res. Tox., 13, 541-549) ou bien par CLHP couplée à une détection électrochimique (Pouget et coll, 2000, Chem. Res. Tox., 13, 541-549).

Selon un autre mode de mise en oeuvre avantageux dudit procédé, préalablement à l'étape (c), les formes super enroulées des plasmides obtenus à l'étape (a) sont purifiées, de préférence par centrifugation sur gradient de saccharose et/ou sur gradient de chlorure de césium.

Selon un autre mode de mise en oeuvre avantageux dudit procédé, également préalablement à l'étape (c), chacun des plasmides de la gamme de plasmides est dilué à une concentration comprise entre 5 et 100 µg/ml, dans un tampon de dilution comprenant de préférence un tampon à pH compris entre 6,5 et 8,0, éventuellement associé à un sel et à un tensioactif non ionique ; de préférence, ledit tampon est un tampon phosphate 10 mM ou un tampon SSC, pouvant contenir du NaCl de 0,05 M à 0,5 M.

Les différents plasmides sont préférentiellement déposés à l'aide d'un robot destiné à la fabrication des micro-matrices, c'est-à-dire que les volumes déposés sont compris préférentiellement entre 100 et 1000 picolitres.

Selon un mode de mise en oeuvre avantageux de l'étape (c) dudit procédé, ledit support est un support sensibilisé, de façon à augmenter son affinité pour l'ADN, sélectionné dans le groupe constitué par des matériaux organiques ou inorganiques choisis parmi le verre, le silicium et ses dérivés et les polymères synthétiques ou non (membranes de nylon ou nitrocellulose), et dont la surface est éventuellement fonctionnalisée ; de préférence, ledit support est constitué par des lames de verre recouvertes de poly-L-lysine qui adsorbent l'ADN ou des lames de verre fonctionnalisées par des groupements epoxy qui forment des liaisons covalentes avec l'ADN.

Si nécessaire, des traitements sont réalisés de façon à augmenter la fixation de l'ADN sur son support. Ces traitements ne doivent pas créer de lésions supplémentaires sur l'ADN déposé.

Un support type selon l'invention comportant des zones A₁ à Aₓ, chaque zone comprenant l'ensemble de la gamme de plasmides comprend dans chacune desdites zones :
- au moins un dépôt de plasmide témoin et
- un dépôt de plasmide contenant des photoproduits, et/ou
- un dépôt de plasmide contenant des dommages oxydatifs, et/ou
- un dépôt de plasmide contenant des éthéno-bases, et/ou
- un dépôt de plasmide contenant des cassures de l'ADN, et/ou
- un dépôt de plasmide contenant des adduits d'agent cancérigène.

Conformément à l'étape (d) du procédé selon l'invention :
- l'extrait biologique peut être préparé à partir du milieu biologique, selon le procédé de Manley et coll., 1983, Methods Enzymol. 101, 568-582 ou selon le procédé de Biade et coll., J. Biol. Chem, 1998, 273, 898-902, ou selon tout autre procédé susceptible de fournir un milieu contenant des protéines de la réparation.
- le marqueur est sélectionné parmi les molécules d'affinité, les composés fluorescents, des anticorps ou de la biotine ; de préférence, le marqueur ou le révélateur du marqueur est notamment choisi dans le groupe constitué par des composés fluorescents à fluorescence directe (Cy-3 ou le Cy-5) ou indirecte (biotine ou la digoxigénine).
- le support est incubé ensuite à une température favorisant la réaction de réparation de préférence à 30°C pendant un temps compris entre une et cinq heures, de préférence pendant trois heures.

Selon un mode de mise en oeuvre avantageux de l'étape (e) du procédé selon l'invention, le support est lavé au moins une fois à l'aide d'une solution saline contenant un tensioactif non ionique, notamment un tampon phosphate 10 mM, contenant du Tween 20, puis est ensuite rincé à l'eau au moins une fois.

Conformément à l'étape (f) du procédé selon l'invention, la mesure du signal est réalisée par une méthode appropriée au marqueur ; par exemple, si le marqueur est un fluorophore, on procède à la mesure directe des signaux fluorescents émis par les différents dépôts du support.

Selon un mode de mise an oeuvre avantageux de l'étape (g) du procédé selon l'invention, lesdits signaux sont quantifiés à l'aide d'un appareil capable d'exciter le marqueur, de préférence un fluorophore et de mesurer le signal émis suite à l'excitation.

La mesure du signal est réalisée par une instrumentation adaptée au support et au marqueur utilisé. On pourra utiliser un scanner pour l'analyse d'image en fluorescence, de préférence aux excitations laser à la longueur d'onde spécifique du marqueur employé.

Selon un mode de mise en oeuvre avantageux de l'étape (h) du procédé selon l'invention, on établit un rapport numérique entre les signaux obtenus avec les plasmides contenant les lésions et le signal obtenu avec le plasmide contrôle situé sur le même support.

Selon l'invention, on obtient ainsi un profil de réparation d'un milieu biologique donné.

Ce profil de réparation peut servir à déterminer les capacités de réparation globale et spécifique d'un milieu, à diagnostiquer une maladie liée à la réparation, à évaluer l'influence d'un traitement physique ou chimique (produit génotoxique, par exemple) sur les capacités de réparation d'un milieu donné.

En conséquence la présente invention a également pour objet l'utilisation du procédé tel que défini ci-dessus :
- pour l'établissement du profil de réparation d'un milieu biologique,
- pour le diagnostic d'une maladie liée à une réparation
- pour évaluer l'influence d'un traitement physique ou chimique sur les capacités de réparation d'un milieu biologique donné
- pour le criblage de substances capables de moduler le système de réparation d'un milieu biologique.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la figure 1 illustre un exemple de configuration du support solide ; on observe un plan de dépôt en neuf zones ;
- la figure 2 représente le plan de dépôt de chaque zone ; et
- la figure 3 représente un diagramme de réparation - cartographie de réparation associée à chaque lignée cellulaire ayant servi à préparer l'extrait utilisé pour la réaction de réparation.
   Il doit être bien entendu, toutefois, que ces exemples sont donnés uniquement à titre d'illustration de l'objet de l'invention, dont ils ne constituent en aucune manière une limitation.

### Exemple 1 : Préparation de la gamme de plasmides et dosage des lésions

Le plasmide pBluescript II est produit pas transformation des cellules « XL1-Blue MRF supercompetent cells » de Stratagène selon le protocole fourni par Stratagène.

Le plasmide est ensuite purifié en utilisant le Qiagen plasmid midi kit, en suivant le protocole préconisé.

### Purification supplémentaire du plasmide

Le plasmide est déposé sur 10 ml de gradient de saccharose 5-20% dans un tampon Tris HCl 25 mM pH 7.5 ; NaCl 1 M ; EDTA 5 mM et centrifugé dans une ultra centrifugeuse Beckman en utilisant un rotor SW-41, à 4°C à 25 000 rpm pendant 18 heures. Des fractions de 1 ml sont ensuite prélevées délicatement et analysées sur gel d'agarose. On ne garde que les fractions contenant au moins 90% de forme enroulée du plasmide. Le plasmide est précipité à l'éthanol et dissous dans du PBS.

### Modifications du plasmide

### - Irradiation UVC - Formation de dimères de pyrimidine de type cyclobutane (CPD) et de photoproduits (6-4)

Le plasmide dilué à 20 µg/ml dans un PBS est irradié en utilisant une lampe germicide Bioblock équipée de deux néons de 15 Watts. Trois préparations de plasmides sont irradiées respectivement à 0,06 ; 0,12 et 0,2 J/cm².

### - Traitement au Chloroacétaldéhyde (CCA-Signa) - Formation de malondialdéhyde-désoxyguanine (MDA-dG)

Au plasmide, préparé à 1 mg/ml dans du PBS, on rajoute un volume équivalent de CAA (50% dans H₂O). On incube cette solution une nuit à 37°C. Le plasmide est récupéré par précipitation et purifié sur gradient de saccharose.

### - Traitement au tran-trans-2, 4decadienal (DDE-Sigma)-Formation d'éthéno-guanosine et d'éthéno-adénosine

A 200 µl de plasmide préparé dans l'eau à 1 mg/ml, on rajoute un volume équivalent de tampon carbonate/bicarbonate 0,2 M pH 9,2 et un volume équivalent de THF. On rajoute alors 4 µl de DDE et 12 µl de H₂O₂ 30%. La solution est incubée 2 heures à 50°C à l'obscurité. Le DDE est éliminé par deux extractions au dichlorométhane. L'ADN est précipité puis purifié sur gradient de saccharose.

### - Traitement avec l'endoperoxyde DHPNO₂ - Formation de 8-oxo-2'désoxyguanosine (8-oxo-dG).

20 µl de la solution d'endoperoxyde (1,4-endoperoxyde de *N,N',-*di(2,3-dihydroxypropyl)- 1,4-naphtalènedipropanamide, préparé selon le protocole décrit dans J. Biol. Chem., 2000, 275, 40601-40604 sont incubés avec 200 µl de plasmide dilué dans du PBS à 1 mg/ml pendant 2 heures à 37°C. Le plasmide est ensuite précipité et purifié sur gradient de saccharose.

### Dosage des lésions dans les plasmides

Une fraction d'ADN plasmidique ou d'ADN de thymus de veau traité dans les mêmes conditions est prélevée pour l'analyse de la composition en bases modifiées.

L'ADN est digéré comme décrit par Douki et al, J. Biol. Chem., 275, 11678-11685, puis l'analyse est réalisée par HPLC-couplée à la spectrométrie de masse en tandem.

On obtient la quantité suivante de lésions pour 10⁴ bases normales :

| Traitement | Etheno Da | Etheno Dg | MDA-Dg | 8-oxo-Dg | Dimère de Pyrimidine | Photoproduit (6-4) |
|---|---|---|---|---|---|---|
| ADN Control | 0.11 | 0.00 | 0.00 | 0.61 | | |
| Endoperoxyde | 0.01 | 0.00 | 0.02 | 6.15 | | |
| DDE | 1.42 | 9.04 | 0.24 | 2.89 | | |
| UVC 0.06 J/cm² | | | | 0.12 | 5.03 | 0.45 |
| UVC 0.12 | | | | 0.18 | 11.08 | 0.93 |
| UVC 0.2 J/cm² | | | | 0.21 | 18.14 | 1.57 |

On constate que les traitements provoquent la formation de lésions dans des proportions très différentes :
- les UVC provoquent très largement la formation de dimères de pyrimidine de type cyclobutane (CPD) et minoritairement de photoproduits (6-4),
- le DDE provoque la formation majoritaire de l'éthéno-désoxyguanosine,
- l'endoperoxide provoque la formation très majoritaire de 8-oxo-2'-désoxyguanosine.

On voit que ces agents permettent d'induire la formation majoritaire de lésions spécifiques ciblant des familles précises d'enzymes de réparation.

### Exemple 2 : mise en oeuvre du procédé selon l'invention avec la gamme de plasmides préparée à l'exemple 1.

### Dépôt des plasmides sur support

Les plasmides sont dilués dans du PBS à 20 µg/ml. On effectue des dépôts de 500 picolitres à l'aide d'un robot GESIM, sur des lames de verre commerciales recouvertes de poly-L-lysine (VWR). Les lames sont conservées à 4°C.

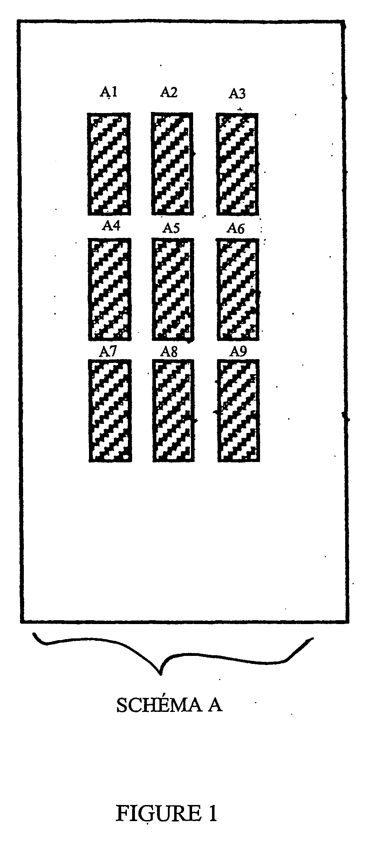

Chaque lame (support S) comporte 9 zones (A1 à A9) identiques disposées selon le schéma A de la figure 1.

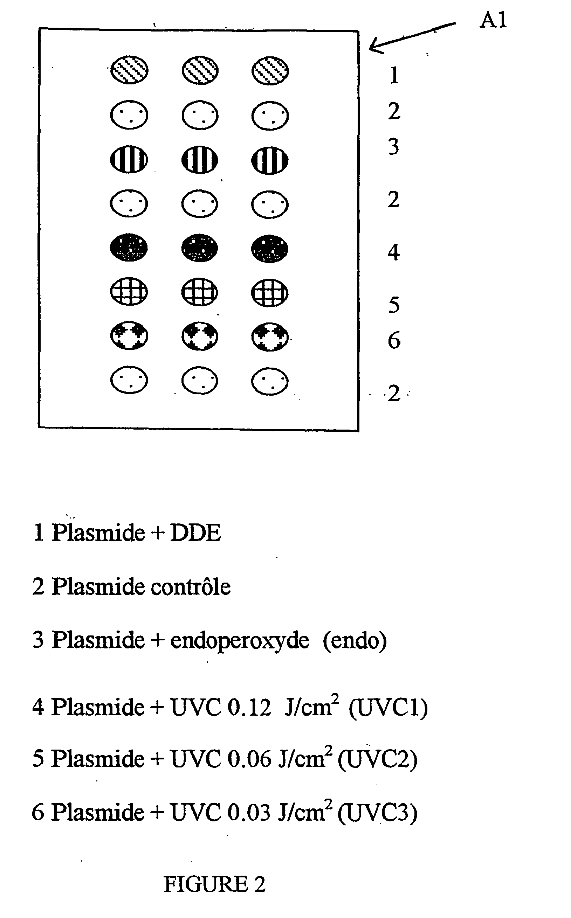

Dans chaque zone, la gamme des plasmides est déposée conformément à la figure 2, qui illustre par exemple la zone A1.

Chaque zone permet de tester un milieu biologique différent.

### Réaction de réparation

Une solution est préparée contenant le milieu ou extrait biologique à tester ; pour 5 µl de solution, la composition est la suivante :
- extrait 0,5 µl
- tampon de réparation 5x 1 µl
- CY5-dUTP (Amersham pharmacia biotech) 0,2 µl (0,1 nmole/µL)
- KCl 2M 0,2 µl
- ATP (Roche - 100mM) 0,1 µl

On complète à 5 µl avec H₂O.

### Composition du tampon de réparation 5x :

Hepes/KOH 220 Mm Ph 7.8 ; MgCl₂ 35 mM; DTT 2,5 mM; dATP

2 µM, dGTP 2 µM; dCTP 2 µM; Phosphocréatine 50 mM; Créatine phosphokinase 250 µg/ml ; BSA 0,5 mg/ml ; glycérol 17%.

### Lignées cellulaires utilisées

L'exemple est réalisé avec trois extraits différents provenant de lignées cellulaires différentes :
. Lignée 1 : il s'agit des cellules HeLa. Les extraits sont des extraits nucléaires commerciaux et proviennent de la société 4C Biotech (Belgique). Ils ont été préparés par la méthode de Dignam et al, (Nucl. Ac. Res., 1983, 11, 1475-1489). Leur contenu en protéines est de 24 mg/ml.
. Lignée 2 : il s'agit d'une lignée de cellules AS203 établies à partir d'un patient atteint de *xeroderma pigmentosum* de groupe de complémentation D. Les extraits ont été préparés d'après le protocole de Manley et al.. Le dosage des protéines par le kit micro BCA kit permet d'évaluer la quantité de protéines à 44 mg/ml.
Lignée 3 : il s'agit de cellules XP12RO. Cette lignée a été établie à partir d'un patient atteint de *xeroderma pigmentosum* de groupe de complémentation A. Les extraits ont été préparés d'après le protocole de Manley et al., (Methods Enzymol., 1983, 101, 568-582). L'extrait obtenu contient 36 mg/ml de protéines (dosage micro BCA kit, Interchim).

On dépose 3 µl de chaque solution de réparation sur l'ensemble des dépôts d'une seule zone de la lame. La lame est incubée à 30°C, à l'humidité, pendant 3 heures. La lame est lavée 3 fois 10 minutes dans un tampon PBS, contenant 0, 1 % de Tween 20. Puis elle est lavée dans H₂O, pendant 15 minutes. Après séchage, on procède à la lecture de fluorescence.

### Analyse des signaux de réparation

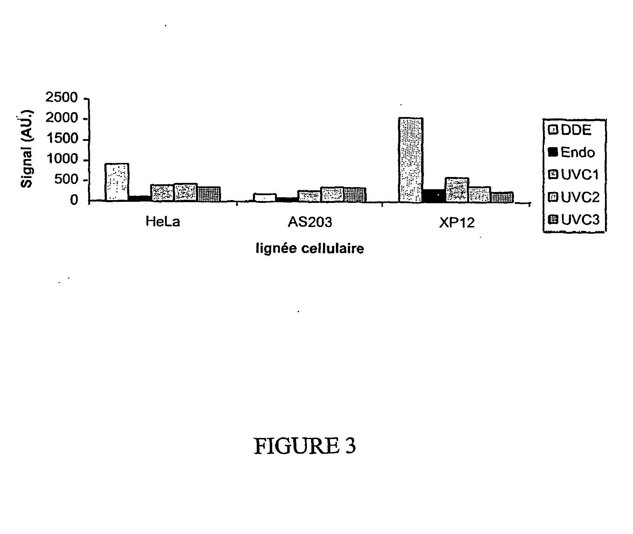

Après la réaction de réparation, la fluorescence des différents dépôts de chaque zone est analysée au moyen d'un scanner Axon à 635 nm et du logiciel d'analyse GenePix Pro. Une moyenne sur trois points identiques est ensuite réalisée. On obtient ainsi une valeur pour chaque type de modification. Un diagramme est tracé pour chaque lignée cellulaire. Ce diagramme correspond à une cartographie des systèmes de réparation associés aux lésions présentes sur le support ou puce et est spécifique de l'extrait utilisé. Les résultats obtenus sont présentés à la figure 3. Le niveau de fluorescence est donné en Unités Arbitraires (AU).

On observe que chaque diagramme est unique et spécifique de la lignée cellulaire ayant servi à préparer l'extrait cellulaire utilisé. Il peut donc servir à caractériser précisément les activités globales de réparation d'un extrait cellulaire donné et est révélateur de la fonctionnalité des systèmes ciblés.

On observe que la lignée HeLa répare deux fois plus efficacement les lésions induites par le DDE (majoritairement étheno-dG) que par les UV (majoritairement CPD et (6-4)). On observe que les dommages oxydatifs (majoritairement 8-oxo-dG) sont réparés beaucoup plus faiblement.

Pour la lignée AS203, on observe un niveau de réparation le plus élevé, bien que faible, pour les lésions UV-induites.

En ce qui concerne la lignée XP12RO, on observe que ce sont les lésions induites par le DDE qui sont réparées le plus efficacement (majoritairement éthéno-dG), donnant un signal trois fois plus élevé que dans le cas de UV C. On sait que les lignées XPA ne réparent pas les CPD ; on peut ainsi attribuer les signaux obtenus avec l'ADN irradié en UVC, à la réparation de photoproduits (6-4).

Un avantage inattendu de l'invention est que, même si la quantité de protéine est différente d'un extrait à l'autre, le rapport obtenu entre les signaux des ADN comportant les lésions et l'ADN contrôle pour un extrait donné, peut être utilisé pour comparer les capacités de réparation des différents extraits entre eux.

## Revendications

1. Procédé d'évaluation quantitative des capacités globales et spécifiques de réparation de l'ADN d'au moins un milieu biologique, lequel procédé est **caractérisé en ce qu'**il comporte les étapes suivantes :
(a) la préparation d'une gamme de plasmides comportant chacun des lésions distinctes de l'ADN, par traitement indépendant desdits différents plasmides par au moins un agent physique et/ou chimique et récupération de la fraction super enroulée de chacun desdits plasmides,
(b) la caractérisation des lésions présentes sur chacun des plasmides de ladite gamme de plasmides,
(c) le dépôt des différents plasmides de ladite gamme de plasmides et d'au moins un plasmide super enroulé contrôle sans lésions sur un support solide unique, selon un schéma A préétabli, pour former un support fonctionnalisé divisé en zones distinctes A₁ à Aₓ, x correspondant à un nombre entier égal au nombre de milieux biologiques à tester simultanément, chaque zone A₁ à Aₓ comprenant ladite gamme de plasmides,
(d) l'incubation dudit support fonctionnalisé obtenu à l'étape (c) avec différentes solutions de réparation comprenant chacune au moins un milieu biologique susceptible de contenir des activités enzymatiques de réparation, de l'ATP, un système de régénération de l'ATP, un nucléotide triphosphate marqué et tout autre composant nécessaire à l'activité des enzymes de réparation présentes dans ledit milieu biologique, de préférence à une température de 30°C pendant 1 à 5 heures, de préférence pendant 3 heures chacune desdites solutions de réparation étant déposée, préalablement à ladite incubation, dans chacune desdites zones A₁ à Aₓ distinctes et préétablies dudit support fonctionnalisé,
(e) au moins un lavage dudit support fonctionnalisé,
(f) la mesure directe ou indirecte du signal produit par le marqueur incorporé dans l'ADN lors de la réaction de réparation de l'étape (d), dans chacune desdites zones A₁ à Aₓ distinctes et préétablies,
(g) l'enregistrement et la quantification du signal correspondant à chaque dépôt de plasmide dans chaque zone A₁ à Aₓ et
(h) la détermination du rapport des signaux des plasmides comportant les lésions par rapport au plasmide contrôle déposé conjointement.

2. Procédé selon la revendication 1, **caractérisé en ce que** les plasmides selon l'étape (a) sont choisis parmi ceux qui possèdent une forme double-brin super enroulée.

3. Procédé selon la revendication 1 ou la revendication 2, **caractérisé en ce qu'**à l'étape (a) dudit procédé, les différents agents physiques ou chimiques aptes à induire une lésion de l'ADN sont choisis parmi ceux qui induisent de préférence : la formation d'une lésion unique, la formation d'un nombre limité de lésions ou la formation de différentes lésions appartenant à une même famille.

4. Procédé selon l'une quelconque des revendications 1 à 3, **caractérisé en ce qu'**à l'étape (a) dudit procédé, différents agents sont mis en oeuvre sur chaque plasmide de ladite gamme de plasmides

5. Procédé selon l'une quelconque des revendications 1 à 4, **caractérisé en ce qu'**à l'étape (b) dudit procédé, la caractérisation des lésions comprend (i) le prélèvement d'une fraction de chaque plasmide lésé, (ii) la digestion de chacune desdites fractions par des enzymes libérant les nucléosides de l'ADN, puis (iii) l'analyse du résultat de la digestion en utilisant une combinaison de techniques séparatives couplées à une technique analytique quantitative.

6. Procédé selon la revendication 5, **caractérisé en ce que** la digestion est mise en oeuvre à l'aide d'au moins l'une des enzymes suivantes : la phosphodiestérase de rate de veau, la nucléase P1, la phosphodiestérase de venin de serpent, et la phosphatase alcaline.

7. Procédé selon la revendication 5 ou la revendication 6, **caractérisé en ce que** le résultat de la digestion enzymatique est analysé par l'une des méthodes suivantes : Chromatographie Liquide Haute Performance (CLHP) couplée à la spectrométrie de masse en Tandem, par CLHP couplée à la Chromatographie Gazeuse ou par CLHP couplée à une détection électrochimique.

8. Procédé selon l'une quelconque des revendications 1 à 7, **caractérisé en ce que** préalablement à l'étape (c), les formes super enroulées des plasmides obtenus à l'étape (a) sont purifiées par centrifugation sur gradient de saccharose et/ou sur gradient de chlorure de césium.

9. Procédé selon l'une quelconque des revendications 1 à 8, **caractérisé en ce qu'**également préalablement à l'étape (c), chacun des plasmides de la gamme de plasmides est dilué à une concentration comprise entre 5 et 100 µg/ml, dans un tampon de dilution comprenant de préférence un tampon à pH compris entre 6,5 et 8,0, éventuellement associé à un sel et à un tensioactif non ionique.

10. Procédé selon l'une quelconque des revendications 1 à 9, **caractérisé en ce qu'**à l'étape (c) dudit procédé, les volumes des dépôts de la gamme de plasmides sont compris préférentiellement entre 100 et 1000 picolitres.

11. Procédé selon l'une quelconque des revendications 1 à 10, **caractérisé en ce qu'**à l'étape (c) dudit procédé, ledit support est un support sensibilisé, de façon à augmenter son affinité pour l'ADN, sélectionné dans le groupe constitué par des matériaux organiques ou inorganiques choisis parmi le verre, le silicium et ses dérivés et les polymères synthétiques ou non, et dont la surface est éventuellement fonctionnalisée.

12. Procédé selon la revendication 11, **caractérisé en ce que** ledit support est constitué par des lames de verre recouvertes de poly-L-lysine qui adsorbent l'ADN ou des lames de verre fonctionnalisées par des groupements epoxy qui forment des liaisons covalentes avec l'ADN.

13. Procédé selon la revendication 11 ou la revendication 12, **caractérisé en ce que** ledit support comprend des zones distinctes A₁ à Aₓ, chacune desdites zones comportant :
- au moins un dépôt de plasmide témoin et
- un dépôt de plasmide contenant des photoproduits, et/ou
- un dépôt de plasmide contenant des dommages oxydatifs, et/ou
- un dépôt de plasmide contenant des éthéno-bases, et/ou
- un dépôt de plasmide contenant des cassures de l'ADN, et/ou
- un dépôt de plasmide contenant des adduits d'agent cancérigène.

14. Procédé selon l'une quelconque des revendications 1 à 13, **caractérisé en ce qu'**à l'étape (e) du procédé selon la revendication 1, le support est lavé au moins une fois à l'aide d'une solution saline contenant un tensioactif non ionique, notamment un tampon phosphate 10 mM, contenant du Tween 20, puis est ensuite rincé à l'eau au moins une fois.

15. Procédé selon l'une quelconque des revendications 1 à 14, **caractérisé en ce qu'** à l'étape (f) du procédé selon la revendication 1, la mesure du signal est réalisée par une méthode appropriée audit marqueur.

16. Procédé selon l'une quelconque des revendications 1 à 15, **caractérisé en ce qu'**à l'étape (g) du procédé selon la revendication 1, lesdits signaux sont quantifiés à l'aide d'un appareil capable d'exciter le marqueur et de mesurer le signal émis suite à l'excitation.

17. Procédé selon l'une quelconque des revendications 1 à 16, **caractérisé en ce qu'**à l'étape (h) du procédé, on établit un rapport numérique entre les signaux obtenus avec les plasmides contenant les lésions et le signal obtenu avec le plasmide contrôle situé sur le même support.

18. Utilisation du procédé selon l'une quelconque des revendication 1 à 17, pour l'établissement du profil de réparation d'un milieu biologique donné.

19. Utilisation du procédé selon l'une quelconque des revendications 1 à 17, pour le diagnostic d'une maladie liée à la réparation

20. Utilisation du procédé selon l'une quelconque des revendications 1 à 17, pour évaluer l'influence d'un traitement physique ou chimique sur les capacités de réparation d'un milieu donné.

21. Utilisation du procédé selon l'une quelconque des revendications 1 à 17, pour le criblage de substances capables de moduler le système de réparation d'un milieu biologique.

## Claims

1. A method for the quantitative assessment of the overall and specific DNA repair capacities of at least one biological medium, which method is **characterized in that** it comprises the following steps:
(a) preparing a range of plasmids, each comprising distinct DNA lesions, by independent treatment of said various plasmids with at least one physical and/or chemical agent and recovery of the supercoiled fraction of each of said plasmids,
(b) characterizing the lesions present on each of the plasmids of said range of plasmids,
(c) depositing the various plasmids of said range of plasmids, and at least one supercoiled control plasmid without lesions, onto a single solid support, according to a pre-established configuration A, so as to form a functionalized support divided into different zones A₁ to Aₓ, x corresponding to an integer equal to the number of biological media to be simultaneously tested, each zone A₁ to Aₓ comprising said range of plasmids,
(d) incubating said functionalized support obtained in step (c) with various repair solutions, each comprising at least one biological medium that may contain enzyme activities for repair, ATP, an ATP-regenerating system, a labeled nucleotide triphosphate and any other component necessary for the activity of the repair enzymes present in said biological medium, preferably at a temperature of 30°C for 1 to 5 hours, preferably for 3 hours, each of said repair solutions being deposited, prior to said incubation, in each of said different and pre-established zones A₁ to Aₓ of said functionalized support,
(e) washing said functionalized support at least once,
(f) directly or indirectly measuring the signal produced by the label incorporated into the DNA during the repair reaction in step (d), in each of said different and pre-established zones A₁ to Aₓ,
(g) recording and quantifying the signal corresponding to each deposit of plasmid in each zone A₁ to Aₓ, and
(h) determining the ratio of the signals of the plasmids comprising the lesions relative to the control plasmid jointly deposited.

2. The method as claimed in claim 1, **characterized in that** the plasmids according to step (a) are chosen from those that have a double-stranded supercoiled form.

3. The method as claimed in claim 1 or claim 2, **characterized in that**, in step (a) of said method, the various physical or chemical agents capable of inducing a lesion of the DNA are chosen from those that preferably induce: the formation of a single lesion, the formation of a limited number of lesions or the formation of various lesions belonging to the same family.

4. The method as claimed in any one of claims 1 to 3, **characterized in that**, in step (a) of said method, various agents are used on each plasmid of said range of plasmids.

5. The method as claimed in any one of claims 1 to 4, **characterized in that**, in step (b) of said method, the characterization of the lesions comprises (i) taking a fraction of each plasmid with lesions, (ii) digesting each of said fractions with enzymes that release the nucleosides from the DNA, and then (iii) analyzing the result of the digestion using a combination of separative techniques coupled to a quantitative analytical technique.

6. The method as claimed in claim 5, **characterized in that** the digestion is carried out using at least one of the following enzymes: calf spleen phosphodiesterase, P1 nuclease, snake venom phosphodiesterase, and alkaline phosphatase.

7. The method as claimed in claim 5 or claim 6, **characterized in that** the result of the enzyme digestion is analyzed by means of one of the following techniques: high performance liquid chromatography (HPLC) coupled to tandem mass spectrometry, by HPLC coupled to gas chromatography or by HPLC coupled to electrochemical detection.

8. The method as claimed in any one of claims 1 to 7, **characterized in that**, prior to step (c), the supercoiled forms of the plasmids obtained in step (a) are purified by sucrose gradient centrifugation and/or cesium chloride gradient centrifugation.

9. The method as claimed in any one of claims 1 to 8, **characterized in that**, also prior to step (c), each of the plasmids of the range of plasmids is diluted to a concentration of between 5 and 100 µg/ml, in a diluting buffer preferably comprising a buffer at a pH of between 6.5 and 8.0, optionally combined with a salt and with a nonionic surfactant.

10. The method as claimed in any one of claims 1 to 9, **characterized in that**, in step (c) of said method, the volumes of the deposits of the range of plasmids are preferably between 100 and 1000 picoliters.

11. The method as claimed in any one of claims 1 to 10, **characterized in that**, in step (c) of said method, said support is a support that has been sensitized so as to increase its affinity for the DNA, selected from the group consisting of organic or inorganic materials chosen from glass, silicon and its derivatives, and synthetic or nonsynthetic polymers, and the surface of which is optionally functionalized.

12. The method as claimed in claim 11, **characterized in that** said support consists of glass slides coated with poly-L-lysine that adsorb the DNA, or glass slides functionalized with epoxy groups that form covalent bonds with the DNA.

13. The method as claimed in claim 11 or claim 12, **characterized in that** said support comprises different zones A₁ to Aₓ, each of said zones comprising:
- at least one deposit of control plasmid, and
- a deposit of plasmid containing photoproducts, and/or
- a deposit of plasmid containing oxidative damage, and/or
- a deposit of plasmid containing etheno-bases, and/or
- a deposit of plasmid containing DNA breakages, and/or
- a deposit of plasmid containing carcinogenic agent adducts.

14. The method as claimed in any one of claims 1 to 13, **characterized in that**, in step (e) of the method as claimed in claim 1, the support is washed at least once with a saline solution containing a nonionic surfactant, in particular a 10 mM phosphate buffer containing Tween 20, and is then subsequently rinsed with water at least once.

15. The method as claimed in any one of claims 1 to 14, **characterized in that**, in step (f) of the method as claimed in claim 1, the signal is measured by means of a method suitable for the label.

16. The method as claimed in any one of claims 1 to 15, **characterized in that**, in step (g) of the method as claimed in claim 1, said signals are quantified using a device capable of exciting the label and of measuring the signal emitted subsequent to the excitation.

17. The method as claimed in any one of claims 1 to 16, **characterized in that**, in step (h) of the method, a numerical ratio of the signals obtained with the plasmids containing the lesions to the signal obtained with the control plasmid located on the same support is established.

18. The use of the method as claimed in any one of claims 1 to 17, for establishing the repair profile of a given biological medium.

19. The use of the method as claimed in any one of claims 1 to 17, for diagnosing a repair-related disease.

20. The use of the method as claimed in any one of claims 1 to 17, for assessing the influence of a physical or chemical treatment on the repair capacities of a given medium.

21. The use of the method as claimed in any one of claims 1 to 17, for screening substances capable of modulating the repair system of a biological medium.

## Patentansprüche

1. Verfahren zur quantitativen Bestimmung der allgemeinen und spezifischen Reparaturkapazität von DNA von wenigstens einem biologischem Medium, wobei das Verfahren **dadurch gekennzeichnet ist, dass** es die nachfolgenden Verfahrensschritte umfasst:
(a) Herstellung einer Reihe von Plasmiden, von denen jedes verschiedene Schädigungen der DNA aufweist, durch unabhängige Behandlung der verschiedenen Plasmide mit wenigstens einem physikalischen und/oder chemischen Agens und Rückgewinnung der überspiralisierten Fraktion von jedem der Plasmide,
(b) Charakterisierung der vorliegenden Schädigungen auf jedem der Plasmide der Reihe von Plasmiden,
(c) Abscheiden der verschiedenen Plasmide der Reihe der Plasmide und wenigstens einem gesteuerten überspiralisiertem Plasmid ohne Schädigungen auf einem einheitlichen festen Träger nach einem zuvor festgelegten Schema A zur Bildung eines, in unterschiedliche Bereiche A₁ bis Aₓ unterteilten, funktionalisierten Trägers, wobei x einer ganzen Zahl entspricht, die gleich der Zahl der gleichzeitig zu testenden biologischen Medien ist und wobei jeder der Bereiche A₁ bis Aₓ die Reihe der Plasmide aufweist,
(d) Inkubieren des in Schritt c) erhaltenen funktionalisierten Trägers mit verschiedenen Lösungen zur Reparatur, von denen jede wenigstens ein biologisches Milieu umfasst, das zum Beinhalten enzymatischer Reparaturaktivitäten geeignet ist, ferner ATP, ein Regenerationssystem für ATP, ein markiertes Nucleotidtriphosphat und alle anderen Bestandteile, die für die enzymatische Reparaturaktivität, die in dem biologischen Milieu notwendig sind, bevorzugt bei einer Temperatur von 30°C während 1 bis 5 Stunden, bevorzugt während 3 Stunden, wobei jede der Reparaturlösungen vor dem Inkubieren in einem der verschiedenen und zuvor festgelegten Bereiche A₁ bis Aₓ auf dem funktionalisierten Träger abgelagert wurde,
(e) wenigstens einem Waschen des funktionalisierten Trägers,
(f) direktes oder indirektes Messen des von dem in die DNA eingebrachten Markers erzeugten Signals während der Reparaturreaktion von Schritt d) in jedem der verschiedenen und zuvor festgelegten Bereiche A₁ bis Aₓ,
(g) Registrieren und Quantifizieren des Signals, das einer jeden Abscheidung von Plasmid in jedem der Bereiche A₁ bis Aₓ entspricht und
(h) Bestimmen des Signalverhältnisses der Plasmide, die Schädigungen aufweisen, gemeinsam im Verhältnis zu gesteuert abgeschiedenen Plasmiden.

2. Verfahren gemäß Anspruch 1, **dadurch gekennzeichnet, dass** die Plasmide gemäß Schritt (a) aus denen ausgewählt sind, die eine doppelsträngige, überspiralisierte Form besitzen.

3. Verfahren gemäß Anspruch 1 oder 2, **dadurch gekennzeichnet, dass** in Schritt (a) des Verfahrens die verschiedenen, zur Induktion einer Schädigung der DNA fähigen physikalischen oder chemischen Agenzien aus denen ausgewählt sind, die bevorzugt die Bildung einer einzigen Schädigung, die Bildung einer beschränkten Anzahl von Schädigungen oder die Bildung von unterschiedlichen, zu einer Familie gehörenden Schädigungen, induzieren.

4. Verfahren gemäß einem der Ansprüche 1 bi 3, **dadurch gekennzeichnet, dass** in Schritt (a) des Verfahrens verschiedene Agenzien auf jedes Plasmid der Reihe von Plasmiden angewendet werden.

5. Verfahren gemäß einem der Ansprüche 1 bis 4, **dadurch gekennzeichnet, dass** die Charakterisierung der Schädigungen in Schritt (b) des Verfahrens (i) die Entnahme einer Fraktion von jedem der geschädigten Plasmide umfasst, (ii) den Verdau von jeder der Fraktionen mittels DNA-Nukleosid-freisetzenden Enzymen und dann (iii) Analysieren des Ergebnisses des Verdaus unter Verwendung einer Kombination von mit einem Verfahren der quantitativen Analyse gekoppelten Trennverfahren.

6. Verfahren gemäß Anspruch 5, **dadurch gekennzeichnet, dass** der Verdau mit Hilfe von wenigstens einem der folgenden Enzyme durchgeführt wird: Kalbsmilz-Phosphodiesterase, Nuklease P1, Schlangengift-Phosphodiesterase und alkalischer Phosphatase.

7. Verfahren gemäß Anspruch 5 oder 6, **dadurch gekennzeichnet, dass** das Ergebnis des Enzymverdaus mittels einer der folgenden Methoden analysiert wird: Hochdruckflüssigkeitschromatographie (HPLC) in Tandemkopplung mit einer Massenspektrometrie, mittels einer mit einer Gaschromatographie gekoppelten HPLC oder mittels einer mit einer elektrochemischen Detektion gekoppelten HPLC.

8. Verfahren nach einem der Ansprüche 1 bis 7, **dadurch gekennzeichnet, dass** die in Schritt (a) erhaltenen, überspiralisierten Formen vor Schritt (c) mittels Zentrifugieren mit Saccharosegradienten und/oder Cäsiumchloridgradienten gereinigt werden.

9. Verfahren gemäß einem der Ansprüche 1 bis 8, **dadurch gekennzeichnet, dass** jedes der Plasmide der Reihe der Plasmide vor Schritt (c) in einem Verdünnungspuffer, bevorzugt einen Puffer mit einem pH zwischen 6,5 und 8,0 umfassend, gegebenenfalls in Verbindung mit einem Salz und einem nichtionischen Tensid, auf eine Konzentration zwischen 5 und 100 µg/l verdünnt wird.

10. Verfahren gemäß einem der Ansprüche 1 bis 9, **dadurch gekennzeichnet, dass** die Volumina der Depots der Reihe der Plasmide in Schritt (c) des Verfahrens bevorzugt zwischen 100 und 1000 Picolitern umfassen.

11. Verfahren gemäß einem der Ansprüche 1 bis 10, **dadurch gekennzeichnet, dass** der Träger in Schritt (c) des Verfahrens ein Träger ist, der so sensibilisiert ist dass seine Affinität zu DNA gesteigert ist, ausgewählt aus der Gruppe, bestehend aus organischen oder anorganischen Materialien ausgewählt aus Glas, Silicium und deren Derivaten und den synthetischen und nicht-synthetischen Polymeren, und deren Oberfläche möglicherweise funktionalisiert ist.

12. Verfahren gemäß Anspruch 11, **dadurch gekennzeichnet, dass** der Träger aus Glasschichten gebildet ist, die mit Poly-L-Lysin beschichtet sind, das DNA absorbiert, oder Glasschichten, die mit Epoxidgruppen funktionalisiert sind, welche kovalente Bindungen mit DNA bilden.

13. Verfahren gemäß Anspruch 11 oder 12, **dadurch gekennzeichnet, dass** der Träger verschiedene Bereiche A₁ bis Aₓ umfasst, wobei jeder der Bereiche
- wenigstens ein Depot eines Vergleichs-Plasmids und
- ein Depot mit Photoprodukten enthaltendem Plasmid
- ein Depot mit oxidativen Schäden enthaltendem Plasmid und/oder
- ein Depot mit Ethenobasen enthaltendem Plasmid und/oder
- ein Depot mit Plasmiden mit Brüchen in der DNA und/oder
- ein Depot mit Plasmiden mit Addukten eines kanzerogenen Agens umfasst.

14. Verfahren gemäß einem der Ansprüche 1 bis 13, **dadurch gekennzeichnet, dass** der Träger in Schritt (e) des Verfahrens gemäß Anspruch 1 wenigstens ein Mal mit Hilfe einer Salzlösung gewaschen wird, die nicht-ionisches Tensid enthält, insbesondere einen 10 mMol Phosphatpuffer, der Tween 20 enthält, dann wenigstens ein Mal mit Wasser gewaschen wird.

15. Verfahren gemäß einem der Ansprüche 1 bis 14, **dadurch gekennzeichnet, dass** das Messen des Signals in Schritt (f) des Verfahrens gemäß Anspruch 1 mittels eines für den Marker geeigneten Verfahrens durchgeführt wird.

16. Verfahren gemäß einem der Ansprüche 1 bis 15, **dadurch gekennzeichnet, dass** die Signale in Schritt (g) des Verfahrens gemäß Anspruch 1 mit Hilfe einer Vorrichtung quantifiziert werden, die in der Lage ist den Marker anzuregen und die nach der Anregung emittierten Signale zu messen.

17. Verfahren gemäß einem der Ansprüche 1 bis 16, **dadurch gekennzeichnet, dass** in Schritt (h) des Verfahrens ein Zahlenverhältnis zwischen den mit den Schädigungen aufweisenden Plasmiden erhaltenen Signalen und dem mit dem auf dem selben Träger angeordneten Kontroll-Plasmid erhaltenen Signal festgelegt wird.

18. Verwendung des Verfahrens gemäß einem der Ansprüche 1 bis 17 zur Festlegung eines Reparaturprofils eines gegebenen biologischen Mediums.

19. Verwendung des Verfahrens gemäß einem der Ansprüche 1 bis 17 zur Diagnose einer mit der Reparatur in Zusammenhang stehenden Erkrankung.

20. Verwendung des Verfahrens gemäß einem der Ansprüche 1 bis 17 zur Bestimmung des Einflusses einer physikalischen oder chemischen Behandlung auf die Reparaturkapazität eines gegebenen biologischen Mediums.

21. Verwendung des Verfahrens gemäß einem der Ansprüche 1 bis 17 zum Screening von Substanzen, die zur Modulation des Reparatursystems eines biologischen Mediums in der Lage sind.
